# FASCICULE DE BREVET EUROPEEN

(11) **EP 1 438 015 B1**
(45) Date de publication et mention de la délivrance du brevet: **04.08.2010**
(21) Numéro de dépôt: 02800172.5
(22) Date de dépôt: 01.10.2002
(51) Int. Cl.: A61K 31/409, C07D 487/22

(54) **COMPOSITIONS COSMETIQUES ET DERMOPHARMACEUTIQUES POUR LES PEAUX A TENDANCE ACNEIQUE**
KOSMETISCHE UND DERMOPHARMAZEUTISCHE ZUSAMMENSETZUNGEN FÜR AKNEANFÄLLIGE HAUT
COSMETIC AND DERMOPHARMACEUTICAL COMPOSITIONS FOR SKIN PRONE TO ACNE

(30) Priorité: 03.10.2001 FR 0112802
(43) Date de publication de la demande: 21.07.2004
(73) Titulaire: Sederma, 78612 Le Perray-en-Yvelines Cedex (FR)
(72) Inventeur: LINTNER, Karl, F-78120 Rambouillet (FR)
(74) Mandataire: Laget, Jean-Loup
(86) Numéro de dépôt international: PCT/FR2002/003344
(87) Numéro de publication internationale: WO 2003/028692

(56) Documents cités:
- EP-A- 0 297 733
- EP-A- 1 129 697
- WO-A-01/17523
- DE-A- 10 015 353
- FR-A- 2 785 804
- US-A- 5 723 139
- DATABASE WPI Section Ch, Week 199231 Derwent Publications Ltd., London, GB; Class A96, AN 1992-255651 XP002202868 & JP 04 173739 A (HOKKAIDO TOGYO KK), 22 juin 1992 (1992-06-22)
- PATENT ABSTRACTS OF JAPAN vol. 1997, no. 09, 30 septembre 1997 (1997-09-30) & JP 09 118611 A (NIPPON FLOUR MILLS CO LTD), 6 mai 1997 (1997-05-06)
- DATABASE BIOSIS [en ligne] BIOSCIENCES INFORMATION SERVICE, PHILADELPHIA, PA, US; 2001 GINER-LARZA EVA M ET AL: "Oleanonic acid, a 3-oxotriterpene from Pistacia, inhibits leukotriene synthesis and has anti-inflammatory activity." Database accession no. PREV200100535108 XP002202867 & EUROPEAN JOURNAL OF PHARMACOLOGY, vol. 428, no. 1, 2001, pages 137-143, ISSN: 0014-2999
- MANEZ ET AL: "Effect of selected triterpenoids on chronic dermal inflammation" EUROPEAN JOURNAL OF PHARMACOLOGY, AMSTERDAM, NL, vol. 1, no. 334, 3 septembre 1997 (1997-09-03), pages 103-105, XP002078026 ISSN: 0014-2999
- LIU J: "PHARMACOLOGY OF OLEANOLIC ACID AND URSOLIC ACID" JOURNAL OF ETHNOPHARMACOLOGY, ELSEVIER SCIENTIFIC PUBLISHERS LTD, IE, vol. 2, no. 49, 1 décembre 1995 (1995-12-01), pages 57-68, XP001068332 ISSN: 0378-8741
- SVAMPA MABI: "Oil based cosmetic products extracted from olive leaves" CHEMICAL ABSTRACTS + INDEXES, AMERICAN CHEMICAL SOCIETY. COLUMBUS, US, vol. 66, no. 26, 26 juin 1967 (1967-06-26), page 11041 XP002162942 ISSN: 0009-2258

## Description

Il existe différentes formes d'acné :
➢ Juvénile, qui peut apparaître vers l'âge de 9 ans, sans signe annonciateur de puberté, et qui touche, peu ou prou, environ 70 % des adolescents des deux sexes,
➢ De l'adulte qui, hormis les conséquences d'un traitement hormonal contraceptif, correspond en fait, dans la plupart des cas, à une acné juvénile persistante à la suite d'absence de traitement ou de traitement inapproprié,
➢ Médicamenteuse, dont les principaux responsables sont la vitamine B12, les corticoïdes généraux ou locaux, les préparations à base d'iode ou de brome, les androgènes et les pilules ou progestatifs à tendance androgénique,
➢ Néonatale, qui est due aux hormones androgènes maternelles et qui guérit spontanément,
➢ Excoriée des jeunes filles, qui correspond à l'aggravation de l'acné juvénile normale et classique à cause d'une manipulation quasi obsessionnelle des boutons d'acné. Ainsi, la plupart des acnés dérivent plus ou moins directement de l'acné juvénile. L'acné est vraisemblablement une pathologie multifactorielle qui peut se résumer par le triptyque suivant : hyperséborrhée + trouble de la kératinisation du canal pilo-sébacé + un facteur microbien.

### Hyperséborrhée

Il n'y a pas d'acné sans hyperséborrhée et, grossièrement, l'acné est proportionnelle à l'importance de la séborrhée. La sécrétion de sébum est sous contrôle hormonal, elle constitue même un des meilleurs indices d'imprégnation androgénique, ce qui explique l'apparition de l'acné au moment de la puberté qui correspond à une *explosion* hormonale physiologique.

L'hormone androgénique la plus impliquée dans l'acné est la testostérone.

La testostérone gonadique circule dans l'organisme sous forme liée à une protéine et seule la testostérone libre pénètre dans le cytoplasme de la cellule cible : la glande sébacée. Là, une enzyme, la 5α-réductase, transforme la testostérone en son métabolite actif, la DHT (dihydrotestostérone) qui va stimuler la synthèse des nucléoprotéines. Plus il y a de DHT qui arrive au noyau, et plus les cellules de la glandes sébacées augmentent de taille, se multiplient, se chargent en lipides et, comme il s'agit d'une glande à sécrétion holocrine, plus la quantité de sébum excrété sera importante.

### Trouble de la kératinisation du canal pilo-sébacé

Ce point constitue une condition *sine qua non* de la survenue de l'acné.

L'épithélium qui borde le canal pilo-sébacé au niveau de l'infra-infundibulum, forme alors une grande quantité de cellules kératinisées anormales, une grande quantité de kératine ainsi qu'un cément intercellulaire particulièrement peu soluble. Il se crée au niveau de l'infra-infundibulum un bouchon corné compact qui empêche l'expulsion du sébum: c'est le stade de microkyste ou comédon fermé, véritable lésion élémentaire de l'acné.

Ce microkyste va alors pouvoir évoluer de deux façons :
➢ Soit la kératine et le sébum continuent à gonfler le microkyste et obligent l'acro-infundibulum à se dilater. C'est le comédon ouvert ou banal point noir sans gravité réelle,
➢ Soit il y a rupture de la paroi du microkyste avec irruption dans le derme de sébum, de kératine, d'acides gras libres. Il se forme alors une réaction inflammatoire qui va être aggravée par la dernière composante des causes de l'apparition de l'acné

### Facteur microbien

Il existe à l'état normal dans le follicule pilo-sébacé 3 types de micro-organismes :
➢ Une levure : *pityrosporum ovale* dont le rôle pathogène dans l'acné apparaît nul,
➢ Un staphylocoque blanc non pathogène,
➢ Une bactérie anaérobie, diphtéroïde, Gram + : *corynébactérium acnes* (encore appelé *propionibactérium acnes*) qui synthétise une lipase capable d'hydrolyser les triglycérides du sébum en acides gras libres irritants pour le derme. Ce germe intervient également dans la production locale de protéase, hyaluronidase et neuraminidase qui vont aggraver la situation inflammatoire évoquée au point précédent.

Les données présentées ci-dessus correspondent à un florilège des documents suivants: F. Poli (1996) Acné prépubertaire, Le Concours Médical, 118:905-908; P. Morel (1981) polycopié de dermato et vénéro, Le Kremlin Bicêtre:161-179; François DANIEL (1977) Dermatologie pratique, Edition Dupuy-Compton Médical, Neuilly.

Aussi bien au cours de l'adolescence qu'à l'âge adulte, l'acné provoque chez les personnes qui ont une peau à tendance acnéique, une image de soi négative. L'industrie Cosmétique est donc dans son rôle lorsqu'elle propose une nouvelle approche pour résoudre les problèmes des peaux à tendance acnéique.

Le document US-A-5 723 139 décrit la combinaison de l'acide oléanique avec le rétinole dans des compositions topiques médicales utilisées dans le traitement de l'acné.

Le document FR-A-2 785 804 décrit l'utilisation d'extraits de *Larrea divaricata,* contenant de l'acide nordihydroguaiarétique dans des compositions cosmétiques ou dermopharmaceutiques pour ralentir le vieillissement cutané.

La présente invention a pour but de proposer une composition cosmétique ou dermopharmaceutique efficace destinée à prévenir et traiter les symptômes de l'acné, de l'hyperséborrhée et des peaux à tendance acnéique

Selon l'invention, la composition est **caractérisée en ce qu**'elle contient une quantité efficace et suffisante d'acide oléanolique ou d'un extrait titré de feuilles d'Olea europaea le contenant, et une quantité efficace et suffisante d'acide nordihydroguaiarétique ou d'un extrait titré de feuilles de Larrea divaricata le contenant.

Selon l'invention, l'acide oléanolique ainsi que les extraits de la feuille d'olivier (*Olea europaea)* titrés en acide oléanolique possèdent une forte activité inhibitrice de l'enzyme 5α-réductase et peuvent constituer ainsi un élément important dans la lutte contre les symptômes de la peau acnéique. Il a également été découvert que l'acide oléanolique et les extraits de la feuille d'olivier possèdent une activité antimicrobienne contre *Propionebactérium acnes* et *Acinetobacter calcoaceticus.* Selon l'invention, une synergie est obtenue en associant l'acide nordihydroguaiaretique ou un extrait de *Larrea divaricata* titré en acide nordihydroguaiarétique avec l'acide oléanolique ou les extraits de feuille d'olivier. En effet, l'acide nordihydroguaiarétique est un inhibiteur du métabolisme des protéines dans l'appareil de Golgi et joue ainsi un rôle de modérateur de la croissance cellulaire. L'emploi de l'acide nordihydroguaiarétique pour ralentir la croissance des fanères a été décrite dans FR 2785804. Il s'avère que l'association acide oléanolique et acide nordihydroguaiarétique ou des extraits les contenant renforce l'activité antimicrobienne et diminue l'hyperkératinisation en même temps, ce qui permet de mieux prévenir les symptômes de l'acné.

L'activité anti-acné peut encore être renforcée si l'on formule les compositions cosmétiques et dermopharmaceutiques contenant les extraits cités, en ajoutant d'autres composés appropriés, tels que les actifs hydratants et/ou osmotiques, séborégulateurs, kératolytiques, spécifiquement antimicrobiens et antiinflammatoires.

Les autres constituants des compositions peuvent correspondre à tout ingrédient habituellement ou nouvellement utilisé ou utilisable en Cosmétique ou en Dermopharmacie.

Les exemples 1 à 4 illustrent différentes possibilités de compositions destinées à des utilisations cosmétiques ou dermopharmaceutiques contenant l'acide oléanolique et/ou l'acide nordihydroguaiarétique ou les extraits correspondants. Les exemples 5 à 10 illustrent les effets de ces compositions ainsi que les synergies d'effets constatés avec l'association de différents composants.

### Exemple 1: Mélange concentré (prémix) pour la préparation de produits cosmétiques à visée anti-acné

| | |
|---|---|
| Butylène Glycol | 25 ,00 |
| Acide oléanolique | 0,03 |
| Acide nordihydroguaiarétique | 0,04 |
| PEG-60 Almond glycerides | 10,00 |
| Osmocide^{®} (Sederma) | 64,93 |

### Example 2: Gel nettoyant et hydratant pour peaux à tendance acnéiques

| | |
|---|---|
| Carbopol^{R} 1342 (Goodrich) | 0,3 |
| Propylène glycol | 2 |
| Glycérine | 1 |
| Vaseline blanche | 1,5 |
| Cylomethicone | 6 |
| Alcool cétylique | 0,5 |
| Lubrajel^{®} MS (United Guardian) | 10 |
| Triéthanolamine | 0,3 |
| Acide oléanolique | 0,0015 |
| acide nordihydroguaiarétique | 0,002 |
| Osmocide^{®} (Sederma) | 9,0 |
| Eau, conservateurs, parfum | qsp 100 g |

### Exemple 3: Crème de soin pour peaux grasses

| | |
|---|---|
| Brij^{R} 721 (ICI) | 2,4 |
| Volpo^{R} S72 (Croda) | 2,6 |
| Prostéaryl-15 (Croda) | 8,0 |
| Cire d'abeille | 0,5 |
| Abil^{R} ZP 2434 (Goldschmidt) | 3,0 |
| Propylène glycol | 3,0 |
| Carbopol^{®} 941 (B.F. Goodrich) | 0,25 |
| Triéthanolamine | 0,25 |
| Extrait *d'Olea europaea* | 10,0 |
| Extrait de *Larrea divaricata* | 5.0 |
| Osmocide^{®} (Sederma) | 10,0 |
| Eau, conservateurs, parfums | qsp 100 g |

### Exemple 4 : Lotion nettoyante

| | |
|---|---|
| Ethanol | 1.0 |
| Propylène glycol | 2.0 |
| Abil^{R} B8851 (Goldschmidt) | 0.5 |
| Eumulgin^{®} L (Henkel) | 0.6 |
| Acide oléanolique | 0,05 |
| Eau, conservateurs, parfum | qsp 100 g |

### Exemple 5: Inhibition de la 5a-réductase - dosage enzymatique - in vitro

### Principe et réalisation du test (adaptation selon Zy et Zu - 1998) :

La testostérone est métabolisée de façon irréversible par la 5α-réductase en 5-déhydrotestostérone (5-DHT) en présence de son cofacteur NADPH. La réaction est suivie, à 37 °C, par la mesure, au moyen d'un spectrophotomètre, de la baisse de densité optique du milieu réactionnel à 340 nm, à la suite de la consommation de NADPH en NADP.

Le test consiste donc à mettre en présence de la testostérone, du NADPH et le produit à tester ou de son solvant seul (pour réaliser la réaction témoin).

Après 3 minutes d'attente pour équilibrer le système, la densité optique d'origine du système est mesurée puis, la réaction est déclenché par l'ajout de l'enzyme 5αréductase. Au bout de 10 minutes, une seconde mesure de la densité optique finale est réalisée. La différence entre les deux mesures correspond à la quantité de NADPH consommée, quantité proportionnelle à la quantité de testostérone transformée en 5-DHT.

L'acide oléanolique et l'acide nordihydroguaiarétique ont été testés à différentes concentrations, seuls ou en association.

Résultats: le tableau suivant montre les pourcentages d'inhibitions (moyenne ± SEM) observés au cours de cinq essais indépendants.

| **Produit testé** | **[c]** | **% d'inhibition** |
|---|---|---|
| Acide oléanolique | 0,01 % | 16,7 % ± 1,1 |
| | 0,03 % | 31,7 % ± 2,9 |
| | 0,1 % | 56,3 % ± 2,6 |
| NDGA | 0,01 | 2,1 % ± 1,6 |
| | 0,04 % | 3,7 % ± 2,6 |
| | 0,1 % | 3,0 % ± 1,9 |
| Acide oléanolique + acide nordihydroguaiarétique | 0,03 % | |
| | 0,04 % | 68,3 % ± 3,1 |

➢ Ces résultats démontrent clairement que l'acide oléanolique présente une forte activité inhibitrice de la l'enzyme 5α-réductase puisqu'on observe dans nos conditions une inhibition de 56% à seulement 0,1%. De plus, cet effet est spécifique puisqu'il est dépendant de la concentration.
➢ L'acide nordihydroguaiarétique utilisé seul ne présente aucune activité sur ce test.
➢ Il y a une forte synergie entre les effets lorsque les deux produits sont utilisés en association puisque l'inhibition observée (68 %) est largement supérieure à la somme des effets individuels observés sur le même test aux mêmes concentrations puisque la somme des effets atteint seulement 35.4 %.

### Exemple 6 : Inhibition de la 5α-réductase - détermination HPLC - in vitro

Principe et réalisation du test: le bût de cet essai était de confirmer le résultat précédent mais au moyen d'un moyen analytique HPLC. En effet, contrairement à la 5-DHT, la testostérone absorbe la lumière à la longueur d'onde utilisée (245 nm). La diminution de la surface du pic de testostérone (qui se caractérise dans nos conditions opératoires avec un temps de rétention de 26 minutes) traduit la baisse de cette molécule dans le milieu étudié, et donc reflète l'activité de la 5α-réductase.

Le même type de protocole que dans l'exemple précédent est utilisé mais au lieu de lire les différentes densités optiques, les milieux réactionnels sont analysés par HPLC. Résultats : le tableau suivant montre les pourcentages d'inhibitions (moyenne ± SEM) observés au cours de cinq essais indépendants.

| **Produit testé** | **[c]** | **% d'inhibition** |
|---|---|---|
| Acide oléanolique (obtenu à partir de feuille d'olivier) | 0,01 % | 10,9 % ± 1,4 |
| | 0,03 % | 25,6 % ± 2,0 |
| | 0,1 % | 47,7 % ± 3,8 |
| acide nordihydroguaiarétique (obtenu à partir de *L. divaricata)* | 0,01 | 1,2 ± 0,4 |
| | 0,04 % | 1,0 % ± 0,9 |
| | 0,1 % | 0,9 % ± 0,7 |
| Acide oléanolique +Acide nordihydroguaiarétique | 0,03 % | |
| | 0,04 % | 61,8 % ± 4,2 |

Ces résultats démontrent clairement que :
➢ Les effets observés dans cet exemple sont fortement superposables à ceux obtenus dans l'exemple précédent, ce qui confirme l'hypothèse de départ,
➢ L'acide oléanolique présente une forte activité inhibitrice de la l'enzyme 5α-réductase puisqu'on observe dans nos conditions une inhibition de 48% à seulement 0,1%. L'acide nordihydroguaiarétique utilisé seul ne présente aucune activité sur ce test.
➢ Il y a une synergie évidente entre les effets lorsque les deux produits sont utilisés en association puisque l'inhibition observée (61,8%) est largement supérieure à la somme des effets individuels observés sur le même test aux mêmes concentrations puisque la somme des effets atteint seulement 26,6 %.

### Exemple 7 : Effet anti-inflammatoire - in vitro

Principe et réalisation du test: il est possible d'estimer un niveau d'inflammation par la détermination de la quantité de médiateurs tels que prostaglandines, leucotriènes ou interleukines.

Dans cet exemple, nous avons choisi le dosage de l'interleukine 6 (IL-6) sur des fibroblastes (FH) et sur des kératinocytes (KH) humains normaux en culture, après irradiations par les UV-B.

Schématiquement, les cellules sont mise en culture, dans un milieu de culture classique DMEMc + 10% SVF pendant des périodes de 24 heures pour le FH et d'une semaine pour les KH. Après élimination du tampon et deux rinçages successifs par une solution tampon phosphate, les cellules sont irradiées avec des UV-B sous un niveau d'énergie standardisé à 50 mJ.cm⁻².

Le tampon est alors rapidement éliminé et remplacé par un milieu de culture identique à celui utilisé en début d'expérience mais contenant les extraits à tester aux concentrations pré-requises ou du DMSO pour la série contrôle. Après 24 heures, le milieu de culture est récolté et la détermination de sa concentration en IL-6 est réalisé au moyen d'une méthode Elisa standard.

Une série supplémentaire est réalisée selon le même protocole si ce n'est l'absence d'irradiation UV-B, afin de déterminer le taux de base et de contrôle la stabilité du système étudié. Enfin, les essais sont réalisés en triplicate.

Le tableau suivant montre les résultats (moyenne □SEM des pourcentages de variations par rapport aux tests réalisés sans exposition aux UV) obtenus au cours de 5 essais indépendants.

| Produit testé | [c] | FH **% de variation** | KH **% de variation** |
|---|---|---|---|
| Aucun | - | + 439 % ± 11 | + 450 % ± 15 |
| Acide oléanolique | 0,01 % | + 455 % ± 14 | + 461 % ± 16 |
| | 0,03 % | + 437 % ± 12 | + 444 % ± 14 |
| | 0,1 % | + 441 % ± 18 | + 450 % ± 14 |
| NDGA | 0,01 | + 356 % ± 18 | + 388 % ± 10 |
| | 0,04 % | + 155 % ± 9 | + 201 % ± 10 |
| | 0,1 % | + 87% ± 7 | +91 % ± 14 |
| Acide oléanolique | 0,03 % | | |
| +acide nordihydroguaiarétique | 0,04 % | + 47 % ± 4 | + 58 % ± 4 |

Ces résultats démontrent clairement que:
➢ L'acide nordihydroguaiarétique présente un remarquable effet anti-inflammatoire sur les deux lignées cellulaires étudiées puisqu'on observe dans nos conditions (FH et KH) une inhibition de respectivement 19 % (+ 356% vs + 439%) et de 14% (+ 388% vs + 450%) à seulement 0,01%. De plus, cet effet est spécifique puisqu'il est dépendant de la concentration.
➢ L'acide oléanolique utilisé seul ne présente aucune activité sur ce test.
➢ Sur les deux lignées cellulaires, il y a une synergie évidente entre les effets lorsque les deux produits sont utilisés en association puisque l'on observe respectivement une inhibition de 90 % (+ 47 % vs + 439% sur les FH) et de 87 % (+ 58% vs +450 % sur les KH), ce qui largement supérieur à la somme des effets individuels observés sur le même test aux mêmes concentrations.

### Exemple 8 : Effet antiproliférateur sur kératinocytes - in vitro

Principe et réalisation du test: comme la quantité d'ADN est fixe d'une cellule à l'autre, la mesure de la quantité globale d'ADN correspond à mesurer le nombre de cellules utilisées pour cette mesure. Ce principe permet de ne pas utiliser en routine des méthodologies plus fines mais très lourdes. Différentes techniques ont été développées sur la base de ce protocole dont notamment celle utilisée ici: en se liant à l'ADN, selon une stoechiométrie constante et connu, le fluorophore Hoechst 33258 présente d'une part une fluorescence augmentée mais également un décalage de son spectre d'émission de 492 nm à 458 nm. Par comparaison à des gammes de calibration préalablement établies, le suivi combiné de ces deux paramètres permet de quantifier la quantité d'ADN présente dans les échantillons cellulaires étudiés. Schématiquement, les kératinocytes (KH) humains normaux sont mis en culture, dans un milieu de culture classique DMEMc + 10% SVF pendant une semaine. , en l'absence de produit à tester (série témoin afin de déterminer la prolifération cellulaire de base dans le système étudié) ou en présence des produits à tester, seuls ou en association. Le fluorophore Hoechst 33258 est ajouté en fin de manipulation, avant le prélèvement des aliquotes de cellules pour le dosage.

Les essais sont réalisés en triplicate au cours de trois essais indépendants entre eux. Résultats: le tableau suivant montre les pourcentages d'inhibition (moyenne ± SEM) sur les quantités d'ADN (et donc sur nombre le cellules présentes observées au cours de cinq essais indépendants).

| **Produit testé** | **[c]** | **% d'inhibition** |
|---|---|---|
| Acide oléanolique | 0,01 % | 0,9 % 1,0 |
| | 0,03 % | 1,1 % ± 1,6 |
| | 0,1 % | 0,7 % ± 0,7 |
| NDGA | 0,01 | 10,2 % ± 1,5 |
| | 0,04 % | 22,3 % ± 1,9 |
| | 0,1 % | 29,9 % ± 1,7 |
| Acide oléanolique | 0,03 % | |
| +acide nordihydroguaiarétique | 0,04 % | 41,7 % ± 3,4 |

Ces résultats démontrent clairement que:
➢ L'acide nordihydroguaiarétique inhibe fortement la prolifération des kératinocytes puisqu'on observe dans nos conditions une inhibition de 10 % à seulement 0,01 %. De plus, cet effet est spécifique puisqu'il est dépendant de la concentration.
➢ L'acide oléanolique utilisé seul ne présente aucune activité sur ce test.
➢ Il y a une synergie évidente entre les effets lorsque les deux produits sont utilisés en association puisque l'inhibition observée (41,7 %) est largement supérieure à la somme des effets individuels observés sur le même test aux mêmes concentrations puisque la somme des effets atteint seulement 23,4 %.

### Exemple 9 : Effet anti-bactérien contre le Corynébactérium acnes - in vitro

Principe et réalisation du test : On incorpore dans une gélose (Biomérieux) en surfusion, coulée dans une boite de Pétri, 1 ml du prémix de l'exemple n° 1 ou de ces composants à tester à la concentration équivalente à 5% du prémix. La boite est agitée pour bien homogénéiser le produit dans la gélose. On dépose 10µl d'une suspension de bactéries à étudier diluée respectivement pour contenir environ 10⁴ et 10⁵ bactéries/ml sur la gélose gélifiée , à température ambiante. Ensuite les boites sont incubées pendant 24 et 48 heures à 37°C et observées visuellement.

Les cinq souches *Corynebactérium minutissimum, Propionebacterium acnes, Staphylococcus aureus, Staphylococcus hominis* et *Acinetobactérium calcoaceticus* ont été étudiées.

Résultats : Le tableau suivant indique les activités antimicrobiennes constatées pour les constituants et leur association dans l'excipient cosmétique.

On observe une activité antimicrobienne sélective de l'acide oléanolique contre les germes *P. acnes* et *A. calcoaceticus,* une de l'acide nordihydroguaiarétique contre *A. calcoaceticus* et *S*. *hominis,* une de l'OSMOCIDE^{®} contre *S*. *aureus, S. hominis* et *P. acnès* alors qu'aucun des autres composants ne montre d'effet.

La combinaison des constituants dans le prémix de l'exemple 1 montre l'activité antimicrobienne la plus complète, inactivant la totalité des germes testés.

Les souches étudiées sont représentées dans ce tableau comme:
Souche A ⇐⇒ *Coryne.Minutissimum*
Souche B ⇐⇒ *Staph. hominis*
Souche C ⇐⇒ *Staph. aureus*
Souche D ⇐⇒ *P. Acnes*
Souche E ⇐⇒ *Acinetob.calcoaceticus*

| Souches étudiées | **A** | | **B** | | **C** | | **D** | | **E** | |
|---|---|---|---|---|---|---|---|---|---|---|
| Bacteries/ml | 10⁵ | 10⁴ | 10⁵ | 10⁴ | 10⁵ | 10⁴ | 10⁵ | 10⁴ | 10⁵ | 10⁴ |
| Butylène Glycol+ ac. oleanolique + | | + | + | + | + | + | - | - | - | - |
| BG+ ac.nordihydroguaiaretique | + | + | - | - | + | + | + | - | - | - |
| BG+ eau | + | + | + | + | + | + | + | + | + | + |
| OSMOCIDE | + | + | - | - | - | - | - | - | + | + |
| PEG-60 Almond oil + eau | + | + | + | + | + | + | + | + | + | + |
| Prémix n° 1 à 5% | - | - | - | - | - | - | - | - | - | - |
| Trichlosan (subst. de référence) | - | - | - | - | - | - | - | - | - | - |

Les résultats à 24 H (ci-haut) sont identiques à ceux observés à 48 H.

Lire: croissance bactérienne pour "+", absence de croissance bactérienne pour"-"

### Exemple 10 : Effet anti-acné - in vivo

L'étude a porté sur 30 volontaires, également répartis entre les deux sexes, âgés de 14 à 20 ans, sélectionnés pour leur peau à tendance acnéique, c'est à dire présentant au moins 5 éléments inflammatoires (papules, pustules, nodules) et/ou 10 lésions rétentionnelles (comédon et microkystes).

Le produit testé, gel de l'exemple N°2, a été appliqué pendant 2 mois, matin et soir, après un nettoyage classique du visage. Tout autre produit (médicament topique, cosmétique ou dermatologique) a été proscrit pendant la période du test.

Le comptage et l'observation des lésions ont été réalisés par dermatologue, sur la même surface cutanée du visage préalablement déterminée (20 cm²), avant le traitement (T0), et après 30 (T30) et 60 (T60) jours de traitement. Une diminution significative des lésions et une amélioration nette de l'état du visage ont pu être constatées après ce traitement cosmétique, sans que le produit provoque irritation, oedème ou autre problème d'intolérance.

L'acide oléanolique associé ou non à l'acide nordihydroguaiarétique et/ou les extraits végétaux les contenant sont donc avantageusement utilisés dans la prévention et le traitement des peaux à tendance acnéique, puisque:
➢ L'acide oléanolique diminue l'hyperséborrhée par inhibition de la 5α-réductase, et
➢ l'acide nordihydroguaiarétique (NDGA) produit un effet anti-inflammatoire et anti-prolifération des kératinocytes et réduction de la hyperkératinisation.

Dans les compositions cosmétiques ou dermopharmaceutiques, il peut être avantageux d'associer les extraits cités avec d'autres actifs afin de renforcer leur effet par additivité ou synergie entre les effets de ces différents produits, ou afin d'associer l'effet décrit dans cette demande de brevet avec un autre effet physiologique bénéfique au niveau cutané, des muqueuses, des phanères (cheveux et cuir chevelu), comme par exemple l'OSMOCIDE^{®} (Sederma).

Les concentrations d'acide oléanolique et éventuellement de l'acide nordihydroguaiarétique dans le produit cosmétique ou dermopharmaceutique peuvent varier entre 1 et 10000 ppm (0,0001 et 1% p/p), préférentiellement entre 10 et 1000 ppm (0,001 et 0,1% p/p). En association avec l'acide nordihydroguaiarétique, on emploie préférentiellement 5 à 50 ppm (0,0005 à 0,05 % p/p d'acide oléanolique et 1 à 100 ppm (0,0001 à 0,01 % p/p) d'acide nordihydroguaiarétique. Les concentrations des extraits plus ou moins bruts ou purifiées des feuilles d'olivier et de *L. divaricata* à utiliser dans les produits finis sont fonction des teneurs en acide oléanolique et acide nordihydroguaiarétique respectivement ; les concentrations finales des molécules principalement actives (acide oléanolique et acide nordihydroguaiarétique) doivent être comprises dans les fourchettes citées.

L'acide oléanolique pur ou sous forme d'extrait titré de feuille d'olivier *(Olea europaea),* seul ou en association avec l'acide nordihydroguaiarétique (pur ou sous forme d'un extrait titré de *Larrea divaricata* ) ou combiné avec d'autres actifs, peut être utilisé dans toute forme galénique employée en cosmétique ou dermopharmacie: émulsions H/E et E/H, laits, lotions, pommades, lotions capillaires, shampooings, savons, sticks et crayons, sprays, huiles corporelles, sans que cette liste soit limitative.

L'acide oléanolique pur ou sous forme d'extrait titré de feuille d'olivier *(Olea europaea),* seul ou en association avec l'acide nordihydroguaiarétique (pur ou sous forme d'un extrait titré de *Larrea divaricata* ) ou combiné avec d'autres actifs, peut être utilisé sous forme de solution, de dispersion, d'émulsion, ou encapsulé dans des vecteurs comme les macro-, micro- ou nanocapsules, des liposomes ou des chylomicrons, ou inclus dans des macro-, micro- ou nanoparticules, ou dans des microéponges, ou adsorbé sur des polymères organiques poudreux, les talcs, bentonites et autres supports minéraux.

L'acide oléanolique pur ou sous forme d'extrait titré de feuille d'olivier *(Olea europaea),* seul ou en association avec acide nordihydroguaiarétique (pur ou sous forme d'un extrait titré de *Larrea divaricata* ) peut être combiné avec tout autre ingrédient habituellement utilisé: lipides d'extraction et/ou de synthèse, polymères gélifiants et viscosants, tensioactifs et émulsifiants, tout principe actif hydro- ou liposoluble, extraits végétaux de toute sorte, peptides synthétiques, protéines, vitamines, extraits tissulaires, extraits marins, filtres solaires, antioxydants.

L'acide oléanolique pur ou sous forme d'extrait titré de feuille d'olivier *(Olea europaea),* seul ou en association avec l'acide nordihydroguaiarétique (pur ou sous forme d'un extrait titré de *Larrea divaricata* ) ou combiné avec d'autres actifs, peut être utilisée dans des compositions cosmétiques ou dermopharmaceutiques pour tous les soins de la peau, à visée hydratante, anti-inflammatoire et dans la prévention et le traitement des peaux à tendance acnéique.

L'acide oléanolique pur ou sous forme d'extrait titré de feuille d'olivier *(Olea europaea),* seul ou en association avec l'acide nordihydroguaiarétique (pur ou sous forme d'un extrait titré de *Larrea divaricata* ) ou combiné avec d'autres actifs, peut être incorporée dans des compositions cosmétiques ou dermopharmaceutiques pour la préparation d'un médicament utilisé pour tous les soins de la peau à visée hydratante, anti-inflammatoire et dans la prévention et le traitement des peaux à tendance acnéique.

L'acide oléanolique pur ou sous forme d'extrait titré de feuille d'olivier *(Olea europaea),* seul ou en association avec l'acide nordihydroguaiarétique (pur ou sous forme d'un extrait titré de *Larrea divaricata* ) ou combiné avec d'autres actifs, peut être utilisé pour l'imprégnation de toute sorte de textiles, fibres synthétiques ou naturelles, laines et tout matériaux susceptibles d'être utilisé pour réaliser des vêtements ou sous-vêtements, tissus actifs de toute sorte, lingettes, patchs, compresses, cotons, et coton- tiges, pansements, éponges démaquillantes, masques, et tout support susceptible d'être directement au contact de la peau et du cuir chevelu, pour en permettre une délivrance topique continue.

## Revendications

1. Composition cosmétique ou dermopharmaceutique destinée à prévenir et traiter les symptômes de l'acné, de l'hyperséborrhée et des peaux à tendance acnéique, **caractérisée en ce qu'**elle contient une quantité efficace et suffisante d'acide oléanolique ou d'un extrait titré de feuilles d'*Olea europaea* le contenant, et une quantité efficace et suffisante d'acide nordihydroguaiarétique ou d'un extrait titré de feuilles de *Larrea divaricata* le contenant.

2. Composition selon la revendication 1, **caractérisée en ce que** la quantité d'acide oléanolique est comprise entre 1 et 10000 ppm (0,0001 et 1 % p/p), préférentiellement entre 10 et 1000 ppm (0,001 et 0,1 % p/p).

3. Composition selon la revendication 1 ou 2, **caractérisée en ce que** la quantité d'acide nordihydroguaiarétique est comprise entre 1 et 10000 ppm (0,0001 et 0,01 % p/p), préférentiellement entre 10 et 1000 ppm (0,00 1 et 0,1 % p/p).

4. Composition selon l'une quelconque des revendications 1 à 3, **caractérisée en ce que** la quantité d'acide oléanolique est comprise entre 5 et 50 ppm (0,0005 et 0,05 % p/p) et 1a quantité d'acide nordihydroguaiarétique est comprise entre 1 et 100 ppm (0,0001 et 0,01 % p/p).

5. Composition selon l'une quelconque des revendications 1 à 4, **caractérisée en ce qu'**elle contient en outre au moins un composé choisi parmi les actifs hydratants et/ou osmotiques, séborégulateurs et kératolytiques, antimicrobiens et antiinflammatoires.

6. Composition selon l'une quelconque des revendications 1 à 5, **caractérisée en ce qu'**elle contient en outre au moins un ingrédient choisi parmi les ingrédients habituellement utilisés dans le domaine cosmétique et dermopharmaceutique : lipides d'extraction et/ou de synthèse, polymères gélifiants et viscosants, tensioactifs et émulsifiants, principe actif hydro-ou liposoluble, extraits végétaux, peptides synthétiques, protéines, vitamines, extraits tissulaires, extraits marins, filtres solaires, antioxydants.

7. Composition selon l'une quelconque des revendications 1 à 6, **caractérisée en ce qu'**elle contient de l'OSMOCIDE® (commercialisé par la Société Sederma, France).

8. Composition selon l'une quelconque des revendications 1 à 7, sous toute forme galénique employée en cosmétique ou dermopharmacie : gel, émulsion H/E et E/H, lait, lotion, pommade, lotion capillaire, shampooing, savon, stick, crayon, spray, huile corporelle.

9. Composition selon l'une quelconque des revendications 1 à 8, sous forme de solution, de dispersion, d'émulsion, ou encapsulée dans des vecteurs comme les macro-, micro-ou nanocapsules, des liposomes ou des chylomicrons, ou inclus dans des macro-, micro-ou nanoparticules, ou dans des microéponges, ou adsorbé sur des polymères organiques poudreux, les talcs, bentonites et autres supports minéraux.

10. Composition selon l'une quelconque des revendications 1 à 9, **caractérisée en ce qu'**elle est imprégnée sur un textile, des fibres synthétiques ou naturelles, laines, tous matériaux susceptibles d'être utilisés pour réaliser des vêtements ou sous-vêtements, tissus actifs de toutes sortes, lingettes, patchs, compresses, cotons, coton-tiges, pansements, éponges démaquillantes, masques, ou tout support susceptible d'être directement au contact de la peau et du cuir chevelu, pour en permettre une délivrance topique continue.

11. Utilisation de la composition selon l'une quelconque des revendications 1 à 10 comme agent anti-acné dans la préparation d'un médicament pour prévenir et traiter les symptômes de l'acné, de l'hyperséborrhée et des peaux à tendance acnéique.

12. Utilisation cosmétique de la composition selon l'une quelconque des revendications 1 à 10 pour tous les soins de la peau à visée hydratante, anti inflammatoire, anti microbienne et pour diminuer l'hyperkératinisation.

## Claims

1. A cosmetic or dermopharmaceutical composition intended to prevent and treat symptoms of acne, hyperseborrhea and skins prone to acne, **characterized in that** it contains an effective and sufficient amount of oleanolic acid or a titrated extract of leaves of *Olea europea* containing it, and an effective and sufficient amount of nordihydroguaiaretic acid or of a titrated extract of leaves of *Larrea divaricata* containing it.

2. The composition according to claim 1, **characterized in that** the amount of oleanolic acid is comprised between 1 and 10,000 ppm (0.0001 and 1% w/w), preferentially between 10 and 1,000 ppm (0.001 and 0.1% w/w).

3. The composition according t o claim 1 or 2, **characterized in that** the amount of nordihydroguaiaretic acid is comprised between 1 and 10,000 ppm (0.0001 and 0.01% w/w), preferentially between 10 and 1,000 ppm (0.001 and 0.1% w/w).

4. The composition according to any of claims 1 to 3, **characterized in that** the amount of oleanolic acid is comprised between 5 and 50 ppm (0.0005 and 0.05% w/w) and the amount of nordihydroguaiaretic acid is comprised between 1 and 100 ppm (0.001 and 0.01% w/w).

5. The composition according to any of claims 1 to 4, **characterized in that** it further contains at least one compound selected from moisturizing and/or osmotic, seboregulatory and keratolytic, antimicrobial and anti-inflammatory active substances.

6. The composition according to any of claims 1 to 5, **characterized in that** it further contains at least one ingredient selected from ingredients customarily used in the cosmetic and dermopharmaceutical field: extraction and/or synthesis lipids, gelling and viscosifying polymers, surfactants and emulsifiers, water-soluble or liposoluble active ingredient, plant extracts, synthetic peptides, proteins, vitamins, tissue extracts, marine extracts, sunscreens, antioxidants.

7. The composition according to any of claims 1 to 6, **characterized in that** it contains OSMOCIDE® (marketed by Sederma, France).

8. The composition according to any of claims 1 to 7, in any galenic form used in cosmetics or dermopharmacy: gel, O/W and W/O emulsion, milk, lotion, ointment, capillary lotion, shampoo, soap, stick, pencil, spray, body oil.

9. The composition according to any of claims 1 to 8, in the form of a solution, dispersion, emulsion, or encapsulated in vectors such as macro-, micro- or nano-capsules, liposomes or chylomicrons, or included in macro-, micro- or nano-particles, or in microsponges, or adsorbed on powdery organic polymers, talcs, bentonites and other mineral carriers.

10. The composition according to any of claims 1 to 9, **characterized in that** it is impregnated on a textile, synthetic or natural fibers, wools, any materials which may be used for making clothes or underwear, active tissues of any kinds, towelettes, patches, compresses, cottons, cotton buds, bandages, make-up removal sponges, masks, or any support which may be directly in contact with the skin or the scalp, in order to allow continuous topical delivery thereof.

11. The use of a composition according to any of claims 1 to 10, as an anti-acne agent in the preparation of a drug for preventing and treating the symptoms of acne, of hyperseborrhea and skins prone to acne.

12. The cosmetic use of the composition according to any of claims 1 to 10 for all skin care with a moisturizing, anti-inflammatory, antimicrobial purpose and for reducing hyperkeratinization.

## Patentansprüche

1. Kosmetische oder dermopharmazeutische Zusammensetzung zum Vorbeugen und zum Behandeln der Symptome der Akne, der Hyperseborrhoe und von akneanfälliger Haut, **dadurch gekennzeichnet, dass** sie eine wirksame und ausreichende Menge Oleanolsäure oder einen titrierten Extrakt aus Blättern von *Olea europaea,* der diese enthält, und eine wirksame und ausreichende Menge Nordihydroguajaretsäure oder einen titrierten Extrakt aus Blättern von *Larrea divaricata,* der diese enthält, enthält.

2. Zusammensetzung nach Anspruch 1, **dadurch gekennzeichnet, dass** die Menge Oleanolsäure zwischen 1 und 10000 ppm (0,0001 und 1 % p/p) inklusive ist, vorzugsweise zwischen 10 und 1000 ppm (0,001 und 0,1 % p/p).

3. Zusammensetzung nach Anspruch 1 oder 2, **dadurch gekennzeichnet, dass** die Menge Nordihydroguajaretsäure zwischen 1 und 10000 ppm (0,0001 und 0,01 % p/p) inklusive ist, vorzugsweise zwischen 10 und 1000 ppm (0,001 und 0,1 % p/p).

4. Zusammensetzung nach einem der Ansprüche 1 bis 3, **dadurch gekennzeichnet, dass** die Menge Oleanolsäure zwischen 5 und 50 ppm (0,0005 und 0,05 % p/p) inklusive ist und die Menge Nordihydroguajaretsäure zwischen 1 und 100 ppm (0,0001 und 0,01 % p/p) inklusive ist.

5. Zusammensetzung nach einem der Ansprüche 1 bis 4, **dadurch gekennzeichnet, dass** sie weiterhin mindestens eine Verbindung enthält, die aus den feuchtigkeitsspendenden und/oder osmotischen, hautfettregulierenden und keratolytischen, antimikrobiellen und antientzündlichen Wirkstoffen ausgewählt ist.

6. Zusammensetzung nach einem der Ansprüche 1 bis 5, **dadurch gekennzeichnet, dass** sie weiterhin mindestens einen Inhaltsstoff enthält, der aus den Inhaltsstoffen ausgewählt ist, die üblicherweise auf kosmetischem und dermopharmazeutischem Gebiet verwendet werden: Extraktions- und/oder synthetische Lipide, gelbildende und viskosierende Polymere, Tenside und Emulgatoren, wasser- oder fettlösliches Wirkprinzip, pflanzliche Extrakte, synthetische Peptide, Proteine, Vitamine, Gewebsextrakte, Meeresextrakte, Sonnenfilter, Antioxidantien.

7. Zusammensetzung nach einem der Ansprüche 1 bis 6, **dadurch gekennzeichnet, dass** sie OSMOCIDE® (Vertrieb durch die französische Firma Sederma) enthält.

8. Zusammensetzung nach einem der Ansprüche 1 bis 7 in jeder galenischen Form, die in der Kosmetik oder Dermopharmazie verwendet wird: Gel, Öl/Wasser- und Wasser/Öl-Emulsion, Milch, Lotion, Pomade, Haarlotion, Shampoon, Seife, Stick, Stift, Spray, Körperöl.

9. Zusammensetzung nach einem der Ansprüche 1 bis 8 in Form von Lotion, Dispersion, Emulsion oder eingekapselt in Vektoren wie die Makro-, Mikro- oder Nanokapseln, von Liposomen oder Chylomikronen, oder eingeschlossen in die Makro-, Mikro- oder Nanokapseln, oder in Mikroschwämme, oder absorbiert auf pudrigen organischen Polymeren, Talken, Bentoniten und anderen mineralischen Trägern.

10. Zusammensetzung nach einem der Ansprüche 1 bis 9, **dadurch gekennzeichnet, dass** sie auf einem Textil, synthetischen oder natürlichen Fasern, Wolle, allen Materialien, die zur Herstellung von Bekleidung oder Unterbekleidung verwendbar sind, aktiven Geweben jeder Art, Tüchern, Patchs, Kompressen, Baumwollpads, Baumwollstäbchen, Verbänden, Abschminkschwämmen, Masken oder jedem Träger, der direkt mit der Haut oder der Kopfhaut in Kontakt kommt, imprägniert ist, um eine kontinuierliche topische Abgabe zu ermöglichen.

11. Verwendung der Zusammensetzung nach einem der Ansprüche 1 bis 10 als Anti-Akne-Wirkstoff in der Zubereitung eines Arzneimittels zum Vorbeugen und zum Behandeln der Symptome der Akne, der Hyperseborrhoe und von akneanfälliger Haut.

12. Kosmetische Verwendung der Zusammensetzung nach einem der Ansprüche 1 bis 10 für alle Pflegen der Haut mit dem Ziel der Versorgung mit Feuchtigkeit, der Bekämpfung von Entzündungen, von Mikroben und zur Verringerung der Hyperkeratinisierung.
